(19) Europäisches Patentamt
European Patent Office
Office européen des brevets

(11) EP 2 310 018 B1

(12) **EUROPÄISCHE PATENTSCHRIFT**

(45) Veröffentlichungstag und Bekanntmachung des Hinweises auf die Patenterteilung:
**18.12.2013 Patentblatt 2013/51**

(21) Anmeldenummer: **09772099.9**

(22) Anmeldetag: **20.06.2009**

(51) Int Cl.:
***A61K 31/541*** *(2006.01)* ***A61K 38/15*** *(2006.01)*
***A61P 33/02*** *(2006.01)* ***A61K 9/10*** *(2006.01)*

(86) Internationale Anmeldenummer:
**PCT/EP2009/004475**

(87) Internationale Veröffentlichungsnummer:
**WO 2010/000399 (07.01.2010 Gazette 2010/01)**

(54) **VERWENDUNG VON NIFURTIMOX ZUR BEHANDLUNG DER GIARDIASIS**

USE OF NIFURTIMOX FOR TREATING GIARDIASIS

UTILISATION DU NIFURTIMOX POUR LE TRAITEMENT DE LA GIARDIOSE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **02.07.2008 DE 102008031284**

(43) Veröffentlichungstag der Anmeldung:
**20.04.2011 Patentblatt 2011/16**

(73) Patentinhaber: **Bayer Intellectual Property GmbH**
**40789 Monheim (DE)**

(72) Erfinder:
- **GREIF, Gisela**
  **53424 Remagen (DE)**
- **HARDER, Achim**
  **51109 Köln (DE)**
- **BACH, Thomas**
  **42349 Wuppertal (DE)**
- **PETRY, Gabriele**
  **47809 Krefeld (DE)**
- **KRUEDEWAGEN, Eva-Maria**
  **40789 Monheim (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**EP-A- 0 382 173 US-A- 3 262 930**

- **ESCOBEDO ANGEL A ET AL: "Giardiasis: a pharmacotherapy review." EXPERT OPINION ON PHARMACOTHERAPY AUG 2007, Bd. 8, Nr. 12, August 2007 (2007-08), Seiten 1885-1902, XP009123116 ISSN: 1744-7666**
- **RYAN-GULLAHORN JEAN: "Giardia and effective cattery management for the veterinarian" FELINE PRACTICE, Bd. 28, Nr. 2, März 2000 (2000-03), Seiten 8-9, XP009123223 ISSN: 1057-6614**
- **RAETHER W ET AL: "Nitroheterocyclic drugs with broad spectrum activity." PARASITOLOGY RESEARCH, Bd. 90, Nr. Supplement 1, Juni 2003 (2003-06), Seiten S19-S39, XP002547457 ISSN: 0932-0113 in der Anmeldung erwähnt**
- **HARDER A ET AL: "Mechanisms of action of emodepside" PARASITOLOGY RESEARCH ; FOUNDED AS ZEITSCHRIFT FÜR PARASITENKUNDE, SPRINGER, BERLIN, DE, Bd. 97, Nr. 1, 1. Oktober 2005 (2005-10-01), Seiten S1-S10, XP019345740 ISSN: 1432-1955**
- **MCKELLAR Q A ET AL: "Veterinary anthelmintics: old and new" TRENDS IN PARASITOLOGY, ELSEVIER CURRENT TRENDS, Bd. 20, Nr. 10, 1. Oktober 2004 (2004-10-01), Seiten 456-461, XP004560074 ISSN: 1471-4922**
- **MONZOTE L: "A review of anti-parasitic patents (1988-2008)" RECENT PATENTS ON ANTI-INFECTIVE DRUG DISCOVERY 2008 GB, Bd. 3, Nr. 3, 2008, Seiten 177-191, XP009123144 ISSN: 1574-891X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 310 018 B1

## Beschreibung

Die vorliegende Erfindung betrifft die Verwendung von Nifurtimox zur Behandlung der Giardiose, insbesondere bei Hund und Katze.

Die Wirksamkeit von nitroheterocyclischen Verbindungen gegen Protozoen-Erkrankungen ist bekannt (1).

Zu den Protozoen zählen einkernige Organismen, deren Grundstruktur eine eukaryontische Zelle ist. In der genauen Systematik gibt es jedoch große Unterschiede in der Lebensweise, Morphologie und dem biochemischen Stoffwechsel der einzelnen Stämme, Klassen, Gattungen und Arten. Daher wirken chemische Substanzen je nach ihrem Angriffspunkt und Wirkprinzip üblicherweise nicht gleichermaßen gegen alle Protozoen sondern nur gegen spezielle Gruppen von Protozoen (2,3,4).

Bisher ist die Wirksamkeit von Nifurtimox nur gegen Protozoen-Arten der Gattung Trypanosoma beschrieben worden, e.g *Trypanasoma brucei* und *Trypanosoma cruzi* (5). Trypanosomen besitzen eine Geißel, die am Basalkörper ("Kinetosom") entspringt und in Verbindung mit dem Grundkörper eine undulierende Membran ausbildet. Parasiten mit diesem morphologischen Grundtypen gehören innerhalb der Protozoen-Systematik zur Ordnung der Kinetoplastida. Trypanosomen vermehren sich überwiegend im Blutplasma und werden durch blutsaugende Arthropoden übertragen. Diese Erreger verursachen die Chagas-Erkrankung ("Trypanosoniasis") des Menschen. Nifurtimox ist derzeit fast die einzige Verbindung, die gegen diese Erreger wirksam. Diese Wirksamkeit beruht vermutlich auf der Hemmung der Trypanothione Reduktase, einem speziellen Enzym von Trypanosomen. Dieses Enzym fehlt anderen Protozoen Erregern.

In einer parallel eingereichten Patentanmeldung beschreiben wir die Wirkung von Nifurtimox gegen Trichomonadida.

Bei der Giardiose handelt es sich um eine Infektionskrankheit, die von flagellierten, einzelligen Parasiten der Gattung Giardia verursacht wird. Diese Gattung gehört zur Ordnung der Diplomonadida. Ihr wichtigster Vertreter ist *Giardia lamblia* (syn. *Giardia intestinalis, Giardia duodenalis*). Weltweit sind bis zu 50%, in Mitteleuropa 2-7% der Hunde- und Katzenproben positiv. Eine Infektion führt besonders in sehr jungen Tieren im 1. Lebenshalbjahr zu langanhaltenden Durchfällen und teilweise blutigem Kot verursacht durch eine Entzündung des Duodenums und Jejunums. Chronische Erkrankungen können zu langanhaltenden Wachstumsverzögerungen führen (6).

Für Giardien-Isolate wurden mehr als 7 Genotypen (A-G) beschrieben. Für den Menschen sind Genotyp A + B infektiös, die wiederum auch in Katze, Hund, Biber, Schaf, Kalb, Pferd, Schwein und Affe vorkommen können. Daher gilt die Krankheit als "Zoonose": Der Mensch kann sich durch verunreinigtes Trinkwasser mit Cysten aus Hund und Katze anstecken. Giardia-Infektionen zählen weltweit zu den häufigsten Verursachern der sogenannten "waterborn outbreaks" im Menschen (7).

Die Infektion erfolgt zumeist durch verunreinigtes Trinkwasser oder infektiöse Faeces auf oralem Wege über die Zystenform. Im Darm schlüpfen aus der Zyste sogenannte Trophozoiten. Ein Trophozoit misst 11-17 x 7-11 μm, enthält zwei Kerne und 8 Flagellen (Geißeln). Im Gegensatz zu anderen flagellierten Parasitengruppen dringen Giardien Trophozoiten nicht intrazellulär in Epithelien ein. Trophozoiten verfügen über einen Saugnapf, mit dessen Hilfe sie sich im Darmlumen von Menschen, Affen, Schweinen, Hunden und Katzen an dessen Außenseite verankern können. Dort vermehren sie sich durch Zweiteilung. Ein massiver Befall blockiert und verändert die resorbierende Darmoberfläche und verursacht teils blutige Durchfälle. Im Blinddarm werden erneut Zysten gebildet, die mit einer Präpatenz von 5-16 Tagen nach der Erstinfektion erneut über den Kot ausgeschieden werden. Cysten können über einen Zeitraum von 4-5 Wochen ausgeschieden werden und bleiben für mehrere Wochen infektiös (8,9).

Morphologisch und biochemisch gibt es große Unterschiede zur Ordnung der Kinetoplastiden: Diplomonadiden besitzen 8 Flagellen und zwei Kerne, aber es gibt keine Mitochondrien und keinen Golgi Komplex und innerhalb der Entwicklung sind keine intrazellulären Stadien bekannt. Im Gegensatz zu Trypanosomen besitzen Oberflächenproteine von Giardien keinen GPI Anker (10).

Bei Hund und Katze wird derzeit eine Therapie mit Metronidazol (Clont®, Flagyl®, Elyzol® in einer Dosierung von 12,5-22 mg/kg KGW 2 x täglich über 5 Tage empfohlen. (11). Metronidazol und andere 5-Nitroimidazole werden durch das Enzym Pyruvat-Ferredoxin-Oxidoreduktase zur Bildung freier Nitro-Radikale aktiviert, die dann den parasitären DNA- Metabolismus angreifen. Daher sind auch Ipronidazole (Ipropan®, 126 mg/l Trinkwasser über 7 Tage) sowie Tinidazol (Fasigyn®, 44 mg/kg KGW über 3 Tage) zur Therapie der Giardiose der Hunde geeignet (12,13).

Aus der Gruppe der Benzimidazole werden Mebendazol, Albendazol oder Fenbendazol (oral über 3 Tage) empfohlen. Benzimidazole interferieren mit der Polymerisation der Mikrotubuli, indem sie an die Untereinheit des ß-Tubulins binden. Mikrotubuli sind wichtige Cytoskelettelemente des Parasiten, die in besonderer Weise die Haftscheibe der Trophozoiten stabilisieren.

Im Humanbereich werden auch Antibiotika (z. B. Paramomycin, 25-35 mg/kg/Tag in drei Dosierungen für 7-10 Tage), Quinacrin und Furazolidon (100 mg t.i.d. 7 Tage lang) oder Nitazoxanid (500 mg b.i.d. für 3 Tage) angewendet (14).

Viele dieser Wirkstoflklassen werden seit langer Zeit zur Behandlung der Giardien-Erkrankung angewendet und für die meisten konnte eine Resistenzentwicklung nachgewiesen werden, die zu dokumentierten

Behandlungsmißerfolgen führt. Daher ist die Entwicklung neuer Wirkstoffe und Behandlungskonzepte zwingend notwendig (15).

Überraschenderweise haben wir jetzt die Wirksamkeit von Nifurtimox gegen Giardien gefunden. Diese Wirksamkeit ist bisher nicht beschrieben worden. Die Wirksamkeit richtet sich gegen die Erregerstadien des Darmes und unterbindet die Zystenbildung.

Die Erfindung betrifft daher:

Die Verwendung von Nifurtimox zur Herstellung von Arzneimitteln zur Behandlung von durch Giardien hervorgerufenen Krankheiten.

Nifurtimox ist die Verbindung der Formel (I):

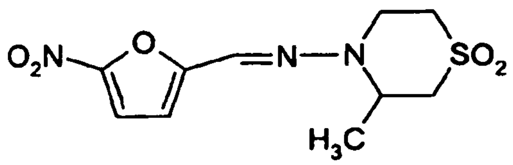

(I)

Gegebenenfalls kommt auch der Einsatz in Form üblicher pharmazeutisch verträglicher Salze in Frage. Weiterhin kommt gegebenenfalls auch der Einsatz von Hydraten oder anderen Solvaten der Wirkstoffe oder gegebenenfalls ihrer Salze in Frage.

Die Anwendung kann sowohl prophylaktisch als auch therapeutisch erfolgen. Die Wirksamkeit richtet sich gegen verschiedene Erregerstadien, insbesondere wirkt Nifurtimox gegen die Erregerstadien des Darmes und unterbindet die Zystenbildung.

Unter den Giardien ist von besonderer Bedeutung *Giardia lamblia* (syn. *Giardia intestinalis, Giardia duodenalis*), *Giardia bovis* und *Giardia caprae.* Die hier aufgeführten Arten werden of als Synonym füreinander aufgeführt und sind wenig wirtspezifisch.

Erfindungsgemäß behandelt werden Tiere (Fleischfresser und Wildtiere), vorzugsweise Säugetiere, wie z. B. Pferd, Schwein, Kaninchen, insbesondere Hunde oder Katzen.

Unter den Säugetieren ist gemäß einer Ausführungsform die Behandlung von Wildtieren und insbesondere Fleischfressern (Hund, Katze) bevorzugt.

Auch der Mensch kommt für eine Behandlung in Betracht, da er sich über kontaminiertes Trinkwasser mit den tierpathogenen Giardia Arten infizieren kann.

Die Giardiose tritt vor allem bei Jungtieren vorzugsweise im Alter von 3-10 Wochen auf und verursacht schwere Durchfälle und eine verminderte Gewichtszunahme.

Die Anwendung der Wirkstoffe erfolgt direkt oder in Form von geeigneten Zubereitungen enteral, parenteral, dermal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht' z.B. in Form des Tauchens (Dippen), Sprühens (Sprayen), Badens, Waschens, Aufgießens (pour-on and spot-on) und des Einpudems. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zur Injektion; Halbfeste Zubereitungen;

Formulierungen, bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden z. B. intravenös, intramuskulär und subcutan verabreicht.

Orale Lösungen werden direkt angewendet. Konzentrate werden nach vorheriger Verdünnung auf die Anwendungskonzentration oral angewendet.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht.

Gele werden auf die Haut aufgetragen oder aufgestrichen oder in Körperhöhlen eingebracht.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Emulsionen sind entweder vom Typ Wasser in Öl oder vom Typ Öl in Wasser und können oral, dermal oder als Injektionen angewendet werden.

Suspensionen können oral, dermal oder als Injektion angewendet werden.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen werden die Wirkstoffe mit geeigneten Trägerstoffen gegebenenfalls unter Zusatz von Hilfsstoffen vermischt und in die gewünschte Form gebracht.

Erfindungsgemäß besonders bevorzugt ist die orale Anwendung, von den üblichen oralen Anwendungsformen sind Tabletten besonders bevorzugt.

Alle oben genannten Arzneimittelformen, die zu verwendenden Zusatz- und Hilfsstoffe sowie die Herstellung dieser Arzneiformen sind dem Fachmann grundsätzlich bekannt.

Die Wirkstoffe können in Kombination mit Synergisten oder mit weiteren Wirkstoffen vorliegen. Als weitere Wirkstoffe seien genannt:

Coccidiosemittel wie Robenidin oder Amprolium z.T. in Kombination mit Folsäureantagonisten (z.B. Pyrimethamin, Epiroprim, Trimetoprim); Antibiotika wie z.B. Clindamycin, Paramomycin oder Spiramycin; Sulfonamide wie z.B. Sulfadimethoxin, Sulfadimidin, Sulfadiazin; Anthelmintika wie z.B. cyclische Depsipeptide (z. B. Emodepsid, PF1022A), Amidinderivate (Tribendimidin, Amidantel, Bay d 9216), Praziquantel oder Benzylbenzoat.

Für einen langfristigen Behandlungsefekt empfiehlt es sich, die Tierhaltung begleitend regelmäßig zu desinfizieren.

Anthelmintika, insbesondere cyclische Oktadepsipeptide wie PF1022A oder Emodepsid eignen sich zur Bekämpfung von Nematoden-Infektionen bei Mensch und Tier (16). Hierbei werden alle ökonomisch bedeutsamen Nematoden im Magen-Darm-Kanal u.a. auch bei Hunden abgetötet. Es ist auch bekannt, dass bei Nicht-Behandlung der Tiere die Immunantwort GALT = gut-associated-lymphoid-tissue) gegen die Würmer gerichtet ist (17). Man spricht hierbei von einer Th2-Antwort des Immunsystems. Normalerweise bei Nichtinfektion ist die Immunantwort ausbalanciert, d.h. der Gegenspieler der Th2-Antwort, nämlich die Th1-Antwort, die gegen Protozoen, Viren und Bakterien gerichtet ist, ist genauso hoch wie die Th2-Antwort.

Liegt im betreffenden Tier, z. B. im Hund, eine Nematoden-Infektion vor, z.B. eine Hakenwurm-, Spulwurm- oder Peitschenwurm-Infektion, so ist die Balance gestört, und es kommt überschießend zu einer Protozoen-Infektion im Darm, hervorgerufen z.B. durch Giardien. Wenn nun durch geeignete Anthelmintika die Nematoden-Infektion geheilt wird, so wird indirekt die Abwehr gegen Giardien verstärkt, indem dann die Th1-Antwort gegen diese Protozoen zur Geltung kommt. In der Kombination Nifurtimox mit Anthelminitika kann dann das Nifurtimox leichter die Giardien-Infektion bekämpfen, da diese bereits teilweise durch die durch die Nematodenbekämpfung indirekt verstärkte Th1-Antwort in Grenzen gehalten wird, d.h die Parasitenzahl im Darm ist bereits vermindert.

Gemäß einer bevorzugten Ausführungsform wird Nifurtimox in Kombination mit Anthelmintika eingesetzt.

Bevorzugt eingesetzte Anthelmintika sind sind 24-gliedrige Cyclodepsipeptide (Cyclooctadepsipeptide). Als solche seien genannt:

Verbindungen der Formel (IIa)

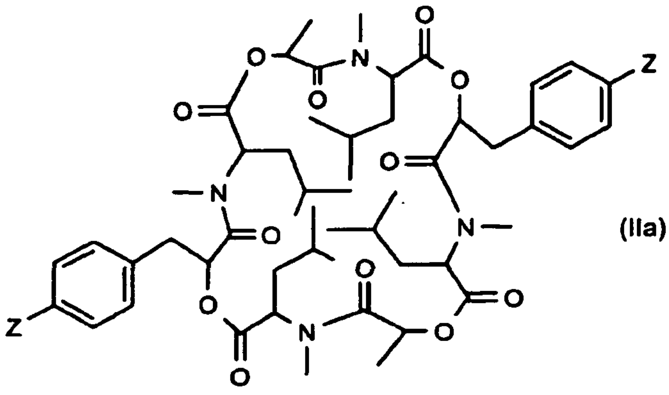

(IIa)

in welcher
Z für Wasserstoff, N-Morpholinyl, $NH_2$, Mono- oder Dimethylamino steht.

Außerdem seien Verbindungen der folgenden Formel (IIb) genannt:

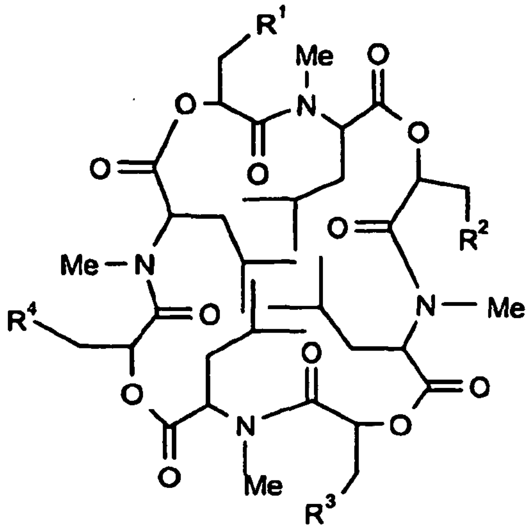

(IIb)

in welcher
$R^1$, $R^2$, $R^3$, $R^4$ unabhängig voneinander für Wasserstoff, $C_1$-$C_{10}$-Alkyl oder Aryl, insbesondere Phenyl stehen, die gegebenenfalls substituiert sind durch Hydroxy, $C_1$-$C_{10}$-Alkoxy oder Halogen.

Die Verbindungen der allgemeinen Formel (IIb) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Zu den cyclischen Depsipeptiden mit 24 Ringatomen zählen auch Verbindungen der allgemeinen Formel (IIc)

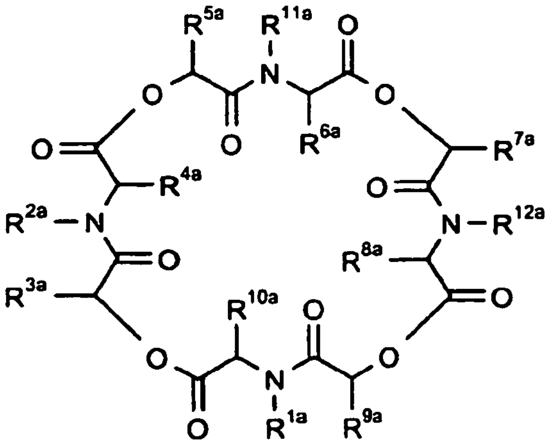

(IIc)

in welcher
$R^{1a}$, $R^{2a}$, $R^{11a}$ und $R^{12a}$ unabhängig voneinander für $C_{1-8}$-Alkyl, $C_{1-8}$-Halogenalkyl, $C_{3-6}$-Cycloalkyl, Aralkyl, Aryl stehen,
$R^{3a}$, $R^{5a}$, $R^{7a}$, $R^{9a}$ unabhängig voneinander für Wasserstoff oder geradkettiges oder verzweigtes $C_{1-8}$-Alkyl steht, das gegebenenfalls durch Hydroxy, $C_{1-4}$-Alkoxy, Carboxy,

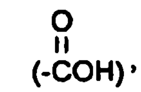

Carboxamid,

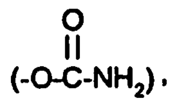

Imidazolyl, Indolyl, Guanidino, -SH oder $C_{1-4}$-Alkylthio substituiert sein kann und ferner für Aryl oder Aralkyl die durch Halogen, Hydroxy, $C_{1-4}$-Alkyl, $C_{1-4}$-Alkoxy substituiert sein können, steht,
$R^{4a}$, $R^{6a}$, $R^{8a}$, $R^{10a}$ unabhängig voneinander für Wasserstoff, geradkettiges $C_{1-5}$-Alkyl, $C_{2-6}$-Alkenyl, $C_{3-7}$-Cycloalkyl, die gegebenenfalls durch Hydroxy, $C_{1-4}$-Alkoxy, Carboxy, Carboxamid, Imidazolyl, Indolyl, Guanidino, SH oder $C_{1-4}$-Alkylthio substituiert sein können, sowie für Aryl oder Aralkyl die durch Halogen, Hydroxy, $C_{1-4}$-Alkyl, $C_{1-4}$-Alkoxy substituiert sein können, stehen, sowie deren optische Isomere und Racemate.

Bevorzugte Verbindungen der Formel (IIc) sind solche, worin
$R^{1a}$, $R^{2a}$, $R^{11a}$ und $R^{12a}$ unabhängig voneinander für Methyl, Ethyl, Propyl, Isopropyl, n-, s-, t-Butyl oder Phenyl, das gegebenenfalls substituiert ist durch Halogen, $C_{1-4}$-Alkyl, OH, $C_{1-4}$-Alkoxy, sowie für Benzyl oder Phenylethyl stehen, die gegebenenfalls durch die bei Phenyl angegebenen Reste substituiert sein können;
$R^{3a}$ bis $R^{10a}$ die oben angegebene Bedeutung haben.

Besonders bevorzugte Verbindungen der Formel (IIc), sind solche, worin
$R^{1a}$, $R^{2a}$, $R^{11a}$ und $R^{12a}$ unabhängig voneinander für Methyl, Ethyl, Propyl, Isopropyl oder n-, s-, t-Butyl stehen,
$R^{3a}$, $R^{5a}$, $R^{7a}$, $R^{9a}$ für Wasserstoff, geradkettiges oder verzweigtes $C_{1-8}$-Alkyl, insbesondere Methyl, Ethyl, Propyl, i-Propyl, n-, s-, t-Butyl, die gegebenenfalls durch $C_{1-4}$-Alkoxy, insbesondere Methoxy, Ethoxy, Imidazolyl, Indolyl oder $C_{1-4}$-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.
$R^{4a}$, $R^{6a}$, $R^{8a}$, $R^{10a}$ unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können, sowie für Isopropyl, s-Butyl ferner für gegebenenfalls halogensubstituiertes Phenyl, Benzyl oder Phenylethyl stehen.

Die Verbindungen der Formel (IIc) können ebenfalls nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Als ganz besonders bevorzugtes Depsipeptid sei die aus EP-OS 382 173 bekannte Verbindung PF 1022, es handelt sich um die Verbindung der Formel (IIa), worin beide Substituenten Z für Wasserstoff stehen. PF 1022 hat daher die folgende Formel (IId):

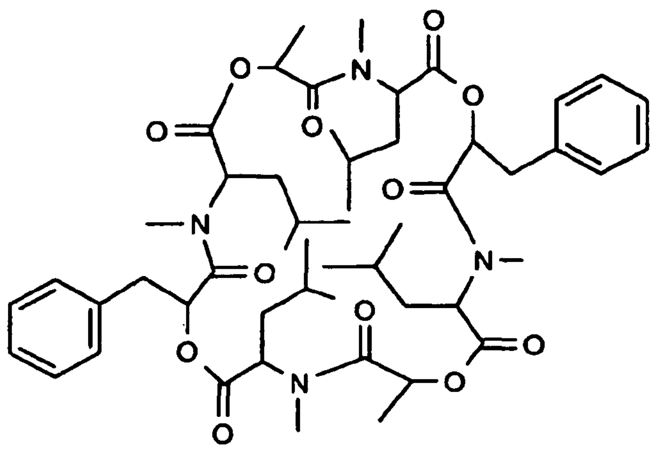

(IId)

Weitere bevorzugte Depsipeptide sind Verbindungen, die aus der PCT-Anmeldung WO 93/19053 bekannt sind, und zwar Verbindungen der Formel (IIa),
in welcher
Z für N-Morpholinyl, $NH_2$, Mono- oder Dimethylamino steht.

Von diesen Verbindungen ganz besonders bevorzugt ist das Depsipeptid Emodepside (PF 1022-221). Es handelt sich um die Verbindung der Formel (IIa), worin beide Reste Z für den Morpholinylrest stehen. Der INN Emodepside steht für die Verbindung mit dem systematischen Namen: Cyclo[(*R*)-lactoyl-*N*-methyl-L-leucyl-(*R*)-3-(*p*-morpholinophenyl)lactoyl-*N*-methyl-L-leucyl-(*R*)-lactoyl-*N*-methyl-L-leucyl-(*R*)-3-(*p*-morpholinophenyl)lactoyl-*N*-methyl-L-leucyl. Emodepside ist in WO 93/19053 beschrieben und hat die folgende Formel:

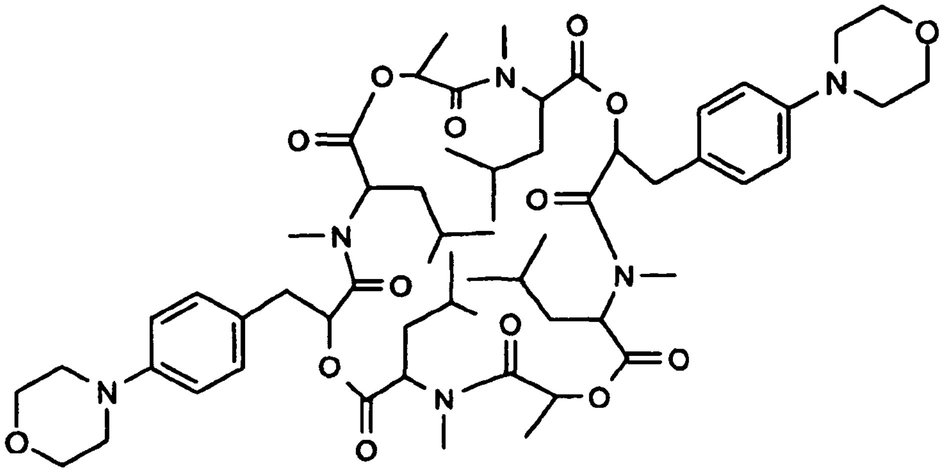

Die vorstehend genannten zur Kombination geeigneten Wirkstoffe können je nach Struktur in stereoisomeren Formen oder als Stereoisomerengemische vorliegen, z.B. als Enantiomere oder Racemate. Sowohl die Stereoisomerengemische als auch die reinen Stereoisomeren können erfindungsgemäß verwendet werden.

Weiterhin können gegebenenfalls verwendet werden: Salze der Wirkstoffe mit pharmazeutisch annehmbaren Säuren oder Basen und auch Solvate, insbesondere Hydrate, der Wirkstoffe oder ihrer Salze.

Die Anwendung in Kombination bedeutet entweder, dass Nifurtimox und der zweite Wirkstoff, insbesondere ein Cyclodepsipeptid, getrennt oder zeitlich abgestuft angewendet werden können. In diesem Fall sind Nifurtimox und der zweite Wirkstoff jeweils als gesonderte Arzneimittel formuliert.

Auch denkbar ist die gleichzeitige Anwendung. Gemäß einer für diesen Fall geeigneten Ausführungsform sind die Wirkstoffe der Kombination gemeinsam in einem Mittel formuliert.
Anwendungsfertige Zubereitungen enthalten den jeweiligen Wirkstoff üblicherweise in Konzentrationen von 10 ppm bis 20 Gew.-%, bevorzugt von 0,1 bis 10 Gew.-%.

Zubereitungen die vor Anwendung verdünnt werden, enthalten den jeweiligen Wirkstoff in Konzentrationen von 0,5 bis 90 Gew.-%, bevorzugt von 5 bis 50 Gew.-%. In konzentrierten Lösungen zur Eindosierung ins Trinkwasser liegt der jeweilige Wirkstoff beispielsweise in Konzentrationen von 0,5 bis 20 Gew.-%, vorzugsweise 1 bis 15 Gew.-%, besonders bevorzugt 2 bis 10 Gew.-% vor.

Im Allgemeinen hat es sich als vorteilhaft erwiesen, Mengen von etwa 0,05 bis etwa 400 mg, bevorzugt 0,1 bis 200 mg, Wirkstoff je kg Körpergewicht pro Tag zur Erzielung wirksamer Ergebnisse zu verabreichen.

In der Mischung mit anderen Coccidiosemitteln, Antibiotika oder Anthelmintika liegen die erfindungsgemäßen Wirkstoffe im Verhältnis 1 zu 0,01 - 50 bis 1 zu 1 - 50 vor.

Die Wirkstoffe können auch zusammen mit dem Futter oder Trinkwasser der Tiere verabreicht werden.

Futter- und Nahrungsmittel enthalten 0,005 bis 1000 ppm, vorzugsweise 0,05 bis 500 ppm des Wirkstoffs in Kombination mit einem geeigneten essbaren Material.

Ein solches Futter- und Nahrungsmittel kann sowohl für Heilzwecke als auch für prophylaktische Zwecke verwendet werden.

Zur Ergänzung der Behandlung mit Nifurtimox kann ein Desinfektionsmittel einegesetzt werden. Mit dem Desinfektionsmittel werden während der Behandlung die Aufenthaltsorte der Tiere (bzw. des Menschen) desinfiziert. Das Desinfektionsmittel sorgt vorzugsweise über die Zerstörung der ausgeschiedenen Cystenformen für eine Eliminierung der parasitären Dauerstadien und verhindert dadurch eine Reinfektion nach Behandlungsende. Das Desinfektionsmittel kann daher schon vor der Behandlung mit Nifurtimox eingesetzt werden, besser ist es jedoch in der Regel es zeitgleich oder zumindest vor Ende der Behandlung mit Nifurtimox einzusetzen.

Desinfektionsmittel sind z. B. solche auf Basis von bioziden Phenolen und/oder Phenolderivaten. Unter bioziden Phenolen werden solche Phenolverbindungen verstanden, die eine freie OH-Gruppe tragen und eine biozide Wirkung aufweisen. Diese Phenole können weitere Ringsubstituenten tragen, wie z.B. Halogene, insbesondere Chlor, $C_{1-6}$-Alkyl, $C_{3-6}$-Cycloalkyl, Phenyl, Chlorphenyl, Benzyl und/oder Chlorbenzyl.

Nicht chlorierte biozide Phenole sind z.B.: 2-Methylphenol, 3-Methylphenol, 4-Methylphenol, 4-Ethylphenol, 2,4-Dimethylphenol, 2,5-Dimethylphenol, 3,4-Dimethylphenol, 2,6-Dimethylphenol, 4-n-Propylphenol, 4-n-Butylphenol, 4-n-Amylphenol, 4-n-Hexylphenol, Thymol (5-Methyl-2-Isopropylphenol), 2-Phenylphenol, 4-Phenylphenol, 2-Benzylphenol. Bevorzugt als nicht chloriertes biozides Phenol eingesetzt wird 2-Phenylphenol.

Chlorierte biozide Phenole sind z.B. 4-Chlor-3-methylphenol (PCMC, p-Chlor-m-kresol), 4-Chlor-3-ethylphenol, 2-n-Amyl-4-chlorphenol, 2-n-Hexyl-4-chlorphenol, 2-Cyclohexyl-4-chlorphenol, 4-Chlor-3,5-xylenol (PCMX, p-Chlor-m-xylenol), 2,4-Dichlor-3,5-xylenol (DCMX, Dichlor-p-xylenol), 4-Chlor-2-phenylphenol, 2-Benzyl-4-chlorphenol, Benzyl-4-chlor-m-kresol, 4-Chlorbenzyl-dichlor-m-kresol. Bevorzugte chlorierte biozide Phenole sind 2-Benzyl-4-chlorphenol, 4-Chlor-3,5-xylenol, 2,4-Dichlor-3,5-xylenol sowie insbesondere 4-Chlor-3-methylphenol.

Unter Phenolderivaten werden hier solche vom Phenol abgeleitete Verbindungen verstanden, deren OH-Gruppe derivatisiert ist, sodass sie keine freie OH-Gruppe enthalten. Bevorzugt sind dies Phenolether, insbesondere mit aliphatischen Alkoholen mit 1 bis 6 Kohlenstoffatomen. Als bevorzugtes Beispiel sei Phenoxyethanol genannt.

Bevorzugt werden die in WO 2007/009606 beschriebenen Desinfektionsmittel eingesetzt, die Biozide und ein Keratolytikum enthalten. Geeignete Biozide oder Biozidkombinationen und geeignete Keratolytika sind im Detail in WO 2007/009606 beschrieben, auf dieses Dokument wird ausdrücklich hingewiesen.

## Beispiele

### Formulierungsbeispiele

Beispiel 1 (Flüssigformulierung):

Suspensionen in 100 ml Glycerinformal/Glycerin-Polyethylenglykolricinoleat (Cremophor® EL)/Wasser im Mischungsverhältnis 1 : 10 mit:

- 500 mg Nifurtimox
- 1000 mg Nifurtimox

Beispiel 2 (Flüssigformulierung):

Suspensionen in 100 ml Cremophor® EL/Wasser im Mischungsverhältnis 1 : 5 mit:

- 500 mg Nifurtimox
- 1000 mg Nifurtimox

Beispiel 3 (Feststoffformulierung)

Die Wirkstoffe werden in den unten angegebenen Mengen als Pulver in eine Gelatinekapsel gefüllt:

- 250 mg Nifurtimox

Beispiel 4 (Tablette)

Nifurtimox-Tabletten sind bekannt und z. B. unter dem Handelsnamen Lampit® als Arzneimittel erhältlich.

**Biologische Beispiele**

**Beispiel 1**

10 - 11 Wochen alte Beagle-Welpen, die sich vor Infektion 11 Tage in Tierzentrum akklimatisieren konnten, wurden mit 50.000 Giardia duodenalis Zysten oral infiziert. Die Zysten für die Infektion wurden aus dem Kot Giardien-Zysten ausscheidender Hunde mit Hilfe eines Sukrosegradienten gewonnen und in Bacto-Casitone Medium für nicht mehr als 2 Wochen bei 4 Grad Celsius gelagert. Ab Tag 10 nach der Infektion wurden die Welpen in Einzelkäfigen gehalten um den individuellen täglichen Gesamtkot zu sammeln. Die quantitative Bestimmung der Zystenausscheidung erfolgte in den 4 Tage vor der Behandlung (Tag -3 bis 0) und die Welpen wurden anhand der Zystenausscheidung auf zwei Gruppen randomisiert. Am Tag 0 erfolgte die Behandlung der 7 Welpen der Behandlungsgruppe einmalig mit einer oralen Gabe von 50 mg/kg Nifurtimox (Lampit®), während die 6 Hunde der Kontrollgruppe unbehandelt blieben. Von Tag 1 bis Tag 8 nach der Behandlung wurde die Zystenausscheidung weiter quantitativ bestimmt.
Ergebnisse: Die Wirksamkeit wurde mit folgender Formel berechnet:

$$\text{Wirksamkeit\%} = \frac{\text{Anzahl Zysten der Kontrollgruppe} - \text{Anzahl Zysten der Behandlungsgruppe}}{\text{Anzahl Zysten Kontrollgruppe}} \times 100$$

Die berechnete Wirksamkeit bei einmaliger Gabe von Nifurtimox betrug 90,4%. (siehe Tabelle 1)

Methode: Die quantitative Bestimmung der Zystenausscheidung erfolgte nach einer modifizierten Version von Hewlett (18): 4 g Kot wurden in 100 ml Wasser gelöst, gesiebt und sedimentieren lassen. Das Sediment wurde auf einen 1 M Sukrosegradienten (spez. Gewicht 1,13) geschichtet und die Zysten, die sich nach Zentrifugation an der Sukrose-Wasser-Trennschicht anreicherten, abpipettiert. Nach einem Spülschritt mit anschließender Zentrifugation wurden die Zysten im Pellet gezählt. Dazu wurde ein Aliquot des Pellets unter dem Mikroskop ausgezählt und die Anzahl der Zysten pro g Kot errechnet.

**Tabelle 1: Giardia-Zystenauscheidung vor und nach Behandlung mit Nifurtimox**

| **50 mg/kg Nifurtimox an Tag T0** | | | | | | | | | | | | |
|---|---|---|---|---|---|---|---|---|---|---|---|---|
| **Hund** | **T -3** | **T -2** | **T -1** | **T 0** | **T 1** | **T 2** | **T 3** | **T 4** | **T 5** | **T 6** | **T 7** | **T 8** |
| 9048 | 22402 | 186750 | 189275 | 19681 | 0 | 0 | 0 | 424 | 0 | 0 | 191 | 1621 |
| 9028 | 5259 | 45672 | 45725 | 16419 | 2636 | 180 | 224 | 1451 | 1318 | 4102 | 4615 | 1768 |
| 8343 | 5339 | 28322 | 26163 | 13644 | 2391 | 0 | 0 | 193 | 153 | 6315 | 223 | 508 |
| 8329 | 29625 | 907 | 1306 | 4397 | 504 | 0 | 0 | 590 | 1700 | 3693 | 8881 | 3294 |
| 9040 | 1063 | 923 | 14238 | 8456 | 4928 | 0 | 0 | 0 | 1094 | 2000 | 431 | 517 |
| 9038 | 1385 | 5897 | 2264 | 0 | 152 | 0 | 0 | 0 | 0 | 1201 | 1873 | 14181 |
| 9020 | 3263 | 905 | 788 | 375 | 0 | 0 | 0 | 652 | 137 | 353 | 608 | 0 |
| | | | | | | | | | | | | |
| Σ | 9762 | 38482 | 39966 | 8996 | 1516 | 26 | 32 | 473 | 629 | 2523 | 2403 | 3127 |
| **Infizierte, nicht behandelte Kontrolle** | | | | | | | | | | | | |
| **Hund** | **T -3** | **T -2** | **T -1** | **T 0** | **T 1** | **T 2** | **T 3** | **T 4** | **T 5** | **T 6** | **T 7** | **T 8** |
| 9008 | 65325 | 41925 | 368 | 0 | 450 | 179 | 1183 | 3288 | 4747 | 14232 | 22237 | 3684 |
| 8341 | 21150 | 44988 | 35525 | 3138 | 1332 | 806 | 23399 | 17739 | 27253 | 9321 | 2660 | 12901 |
| 8309 | 7144 | 34613 | 33688 | 4531 | 25050 | 13238 | 5547 | 26106 | 52000 | 5125 | 47365 | 47999 |
| 8327 | 21250 | 21485 | 1350 | 3991 | 5383 | 4520 | 3529 | 17355 | 6145 | 14298 | 25331 | 110846 |
| 8313 | 2975 | 3185 | 8168 | 1209 | 609 | 6133 | 8438 | 6739 | 18113 | 4027 | 7103 | 2645 |
| 9026 | 945 | 2813 | 1723 | 2038 | 3544 | 3366 | 20163 | 11291 | 6719 | 4621 | 5867 | 5621 |
| | | | | | | | | | | | | |
| Σ | 19798 | 24835 | 13470 | 2485 | 6061 | 4707 | 10377 | 13753 | 19163 | 8604 | 184427 | 30616 |
| T -3 bis T -1: Tage vor der Behandlung<br>T 0: Tag der Behandlung<br>T 1 bis T 8: Tage nach der Behandlung | | | | | | | | | | | | |

| Behandlung | E Giardien-Zystenausscheidung | | Wirksamkeit |
|---|---|---|---|
| | Tag -3 bis 0 | Tag 1 bis 8 | |
| 50 mg/kg Nifurtimox | 24302 | 1341 | 90.4% |
| Infizierte, nicht behandelte Kontrolle | 15147 | 13963 | - |

**Beispiel 2**

11 - 15 Wochen alte Beagle-Welpen, die sich vor Infektion für mindestens 2 Wochen im Tierzentrum akklimatisieren konnten, waren vor Studienbeginn mit 50.000 Giardia duodenalis Zysten oral infiziert worden. Die Zysten für die Infektion waren aus dem Kot Giardien- Zysten ausscheidender Hunde mit Hilfe eines Sukrosegradienten gewonnen und in Bacto-Casitone Medium für nicht mehr als 2 Wochen bei 4 Grad Celsius gelagert worden. 18 Giardien-Zysten ausscheidende Welpen wurden in die Studie eingeschlossen und in Einzelkäfigen gehalten, um den täglichen Gesamtkot zu sammeln. Die quantitative Bestimmung der Zystenausscheidung erfolgte wie in Versuch 145.717, mit der Modifikation daß das Aliquot des Pellets mit Hilfe einer Fuchs-Rosenthal-Zählkammer unter dem Mikroskop gezählt wurde. Die quantitative Bestimmung der Zystenausscheidung erfolgte für 4 Tage vor der Behandlung (Tag -3 bis 0) und die Welpen wurden anhand der Zystenausscheidung auf drei Gruppen randomisiert. An drei aufeinander folgenden Tagen (Tag 0, 1 und 2) erfolgte die Behandlung der 6 Welpen der Behandlungsgruppe 1 morgens mit einer oralen Gabe von 50 mg/kg Nifurtimox (Lampit®), während die 6 Hunde der Behandlungsgruppe 2 an diesen drei Tagen morgens mit einer oralen Gabe von 50 mg/kg Fenbendazol (Fenbendazol-Tab) behandelt wurden. Die 6 Hunde der Kontrollgruppe blieben unbehandelt. Von Tag 1 bis Tag 8 nach der Behandlung wurde die Zystenausscheidung weiter quantitativ bestimmt.
Ergebnisse: Die berechnete Wirksamkeit von Nifurtimox betrug 98,6%, während die Wirksamkeit von Fenbendazol 48,3% betrug. (siehe Tabelle 2)

**Tabelle 2: Giardia-Zystenauscheidung vor und nach Behandlung mit Nifurtimox im Vergleich zur Behandlung mit Fenbendazol.**

| **50 mg/kg Nifurtimox an den Tagen T0, T1 und T2** | | | | | | | | | | | |
|---|---|---|---|---|---|---|---|---|---|---|---|
| **Hund** | **T -3** | **T -2** | **T -1** | **T 0** | **T 1** | **T 2** | **T 3** | **T 4** | **T 5** | **T 6** | **T 7** | **T 8** |
| 9300 | 63463 | 79689 | 91293 | 103442 | 1261 | 527 | 0 | 0 | 0 | 0 | 0 | 903 |
| 0136 | 102900 | 68914 | 59273 | 112084 | 2000 | 0 | 0 | 622 | 1299 | 93 | 0 | 0 |
| 0116 | 57571 | 10685 | 25515 | 60420 | 809 | 741 | 0 | 0 | 229 | 0 | 0 | 517 |
| 8553 | 40909 | 8278 | 3472 | 675 | 187 | 0 | 0 | 185 | 0 | 98 | 0 | 0 |
| 8531 | 4891 | 1618 | 37203 | 89880 | 974 | 0 | 0 | 0 | 0 | 0 | 87 | 975 |
| 0134 | 28773 | 133 | 8528 | 86 | 0 | 0 | 0 | 0 | 2299 | 1842 | 237 | 0 |
| | | | | | | | | | | | | |
| Σ | 49751 | 28220 | 37547 | 61098 | 872 | 211 | 0 | 135 | 638 | 339 | 54 | 399 |
| | | | | | | | | | | | | |
| **50 mg/kg Fenbendazol an den Tagen T0, T1 und T2** | | | | | | | | | | | | |
| **Hund** | **T -3** | **T -2** | **T -1** | **T 0** | **T 1** | **T 2** | **T 3** | **T 4** | **T 5** | **T 6** | **T 7** | **T 8** |
| 0117 | 18781 | 47754 | 243143 | 49400 | 67200 | 0 | 67109 | 49423 | 3116 | 4635 | 17474 | 0 |
| 9320 | 60193 | 23808 | 56014 | 28559 | 14596 | 5980 | 7043 | 652 | 18725 | 7546 | 20780 | 5555 |
| 8555 | 44218 | 26526 | 2349 | 639 | 872 | 0 | 961 | 439 | 4432 | 5594 | 260 | 5968 |
| 8543 | 34119 | 18113 | 16328 | 2719 | 12488 | 3386 | 3075 | 12038 | 0 | 3691 | 18056 | 10875 |
| 0135 | 20463 | 7230 | 14744 | 17037 | 6178 | 490 | 0 | 0 | 0 | 0 | 251 | 0 |
| 9328 | 2003 | 10109 | 21360 | 23290 | 723 | 552 | 5388 | 14133 | 19057 | 11495 | 20657 | 6370 |
| | | | | | | | | | | | | |
| Σ | 29963 | 22257 | 58990 | 20274 | 17010 | 1735 | 13929 | 12781 | 7555 | 5494 | 12913 | 4795 |
| | | | | | | | | | | | | |
| **Infizierte, nichtbehandelte Kontrolle** | | | | | | | | | | | | |

(fortgesetzt)

| Hund | T -3 | T -2 | T -1 | T 0 | T 1 | T 2 | T 3 | T 4 | T 5 | T 6 | T 7 | T 8 |
|---|---|---|---|---|---|---|---|---|---|---|---|---|
| 0121 | 112757 | 27324 | 121092 | 108868 | 153336 | 64620 | 6127 | 190 | 6879 | 85408 | 73978 | 50256 |
| 0138 | 22125 | 65547 | 60200 | 26964 | 22234 | 855 | 17893 | 8138 | 13204 | 3681 | 439 | 1470 |
| 9332 | 22346 | 34475 | 42076 | 24466 | 24360 | 19145 | 12575 | 1455 | 5712 | 16122 | 21440 | 12563 |
| 8529 | 8884 | 49213 | 12881 | 32105 | 71378 | 1139 | 6409 | 4739 | 5297 | 23725 | 11626 | 2835 |
| 9330 | 18829 | 4711 | 22120 | 2476 | 1987 | 0 | 246 | 2492 | 4472 | 602 | 2052 | 813 |
| 0137 | 4623 | 12238 | 14406 | 14328 | 2134 | 10156 | 16756 | 0 | 8170 | 104020 | 79097 | 55935 |
| | | | | | | | | | | | | |
| Σ | 31594 | 32251 | 45463 | 34868 | 45905 | 15986 | 10001 | 2836 | 7289 | 38926 | 31439 | 20645 |

T -3 bis T -1: Tage vor der Behandlung
T 0, T1, T2: Tage der Behandlung
T 3 bis T 8: Tage nach der Behandlung

| Behandlung | Σ Giardien-Zystenausscheidung | | Wirksamkeit |
|---|---|---|---|
| | Tag -3 bis 0 | Tag 3 bis 8 | |
| 50 mg/kg Nifurtimox | 44154 | 261 | 98.6% |
| 50 mg/kg Fenbendazol | 32871 | 9578 | 48.3 % |
| Infizierte, nicht behandelte Kontrolle | 36044 | 18523 | - |

**Literatur**

(1) Raether w., Hänel H. (2003): Nitroheterocyclic drugs with broad spectrum activity Parasitol Res. 90:S19-S39.

(2) Harder A, Greif G, Haberkorn A. (2001a): Chemotherapeutic approoaches to protozoa: Haernosporina - current level of knowledge and outlook.

(3) Harder A, Greif G., Haberkorn A. (2001b): Chemotherapeutic approaches to protozoa: Giardia, Trichomonas and Entamoeba - current level of knowledge and outlook.

(4) Greif G, Harder A, Haberkorn A (2001): chemotherapeutic approaches to protozoa: Caccidiae - current level of knowledge and outlook.

(5) Harder A, Greif G., Haberkorn A (2001c): Chemotherapeutic approaches to protozoa: Kinetoplastida - current level of knowledge and outlook. Parasitol Res 87:778-780.

(6) Adam R.D. (2001): Biology of Giardia lamblia. Clinical Microbiology Reviews, July: 447-475.

(7) Marshall MM, Naumovitz D., Ortega Y., Sterling CR. (1997): Waterborne Protozoan Pathogens. Clinical Microbiology Reviews Jan:67-85.

(8) Roxström-Lindquist, K. Palm D., Reiner D., Ringqvist E., Svärd SG. (2006): Giardia immunity - an update. Trends in Parasitology Vo. 22(1):26-31.

(9) Beckmann L. (2003): Mucosal defences against Giardia. Parasite Immunology 25:259-270.

(10) Hülsmeier A.J., Köhler P. (2005): Giardia duodenalis: direct experimental evidence fort he absence of a glycosylphosphatidylinositol anchor in a variant surface protein. Experimental Parasitology 109:49-52.

(11) Kirkpatrick, CE, Farrell JP (1984): Feline giardiasis: observations on natural and induced infections. Amer. J. Vet. Res. 45:2182-2188.

(12) Zimmer JF, Burrington DB (1986): Comparison of four protocols fort the treatment ofcanine giardiasis. J. Amer. Anim. Hosp. Ass. 22:168-172.

(13) Abbitt B, Huey RL, Eugster AK, Syler J. (1986): Treatment of giardiasis in adult greyhounds, using ipronidazole-medicated water. J. Amer. Vet. Med. Ass. 188:67-69.

(14) Wright JM., Dünn L.A., Upcroft P., Upcroft J.A. (2003): Efficacy of antigiardial drugs. Expert Opin. Drug Saf. 2 (6):529-541.

(15) Escobedo A.A & Cimerman S. (2007): Giardiasis: a pharmacotherapy review. Expert Opin. Pharmacother. 8 (12):1885-1902.

(16) Harder A. et al. (2003) Cyclooctadepsipeptides - an anthelmintically active class of compounds exhibiting a novel mode of action. Int. J. Antimicrobial Agents. 22 : 318-331.

(17) Mehlhorn (ed) (2001) Giardiasis, Man; Encyclopedic Reference of Parasitology, Diseases, treatment, Therappy, Second Edition, Springer-Verlag, pp. 234-235.

(18) Hewlett, E.L., Andrews, J.S.Jr., Ruffier, J., Schaefer III, F.W. (1982): experimental Infection of Mongroel Dogs with Giardia lamblia Cysts and Cultured Trophozoites In: The Journal of infectious diseases, Vol. 145, No. 1, pp 89-93

**Patentansprüche**

1. Verwendung von Nifurtimox zur Herstellung von Arzneimitteln zur Behandlung von durch Giardien hervorgerufenen Krankheiten.

2. Verwendung gemäß Anspruch 1 zur Behandlung von durch *Giardia lamblia* hervorgerufenen Krankheiten.

3. Verwendung gemäß einem der Ansprüche 1 oder 2 zur Behandlung von Katzen oder Hunden.

4. Nifurtimox zur Verwendung zur Behandlung von durch Giardien hervorgerufenen Krankheiten.

5. Nifurtimox zur Verwendung gemäß Anspruch 4 zur Behandlung von durch *Giardia lamblia* hervorgerufenen Krankheiten.

6. Nifurtimox zur Verwendung gemäß einem der Ansprüche 4 oder 5 bei Hunden oder Katzen.

7. Mittel enthaltend Nifurtimox und ein Anthelmintikum.

8. Mittel enthaltend Nifurtimox und ein anthelmintisches Cyclooctadepsipeptid.

9. Mittel gemäß Anspruch 8 enthaltend als anthelmintisches Cyclooctadepsipeptid PF 1022.

10. Mittel gemäß Anspruch 8 enthaltend als anthelmintisches Cyclooctadepsipeptid Emodepsid.

**Claims**

1. Use of nifurtimox for the preparation of medicaments for the treatment of diseases caused by *Giardia* species.

2. Use according to Claim 1 for the treatment of diseases caused by *Giardia lamblia.*

3. Use according to any of Claims 1 or 2 for the treatment of cats or dogs.

4. Nifurtimox for use in the treatment of diseases caused by *Giardia* species.

5. Nifurtimox for the use according to Claim 4 for the treatment of diseases caused by *Giardia lamblia.*

6. Nifurtimox for the use according to any of Claims 4 or 5 in dogs or cats.

7. Composition comprising nifurtimox and an anthelminthic.

8. Composition comprising nifurtimox and an anthelminthic cyclooctadepsipeptide.

9. Composition according to Claim 8, comprising PF 1022 as anthelminthic cyclooctadepsipeptide.

10. Composition according to Claim 8, comprising emodepside as anthelminthic cyclooctadepsipeptide.

**Revendications**

1. Utilisation du nifurtimox pour la fabrication de médicaments destinés au traitement de maladies causées par des

*Giardia.*

2. Utilisation selon la revendication 1 pour le traitement de maladies causées par *Giardia lamblia.*

3. Utilisation selon la revendication 1 ou 2, pour le traitement de chats ou de chiens.

4. Nifurtimox destiné à l'utilisation pour le traitement de maladies causées par des *Giardia.*

5. Nifurtimox destiné à l'utilisation selon la revendication 4 pour le traitement de maladies causées par *Giardia lamblia.*

6. Nifurtimox destiné à l'utilisation selon la revendication 4 ou 5 chez des chiens ou des chats.

7. Composition contenant du nifurtimox et un antihelminthique.

8. Composition contenant du nifurtimox et un cyclo-octadepsipeptide antihelminthique.

9. Composition selon la revendication 8, contenant du PF 1022 en tant que cyclo-octadepsipeptide antihelminthique.

10. Composition selon la revendication 8, contenant de l'émodepside en tant que cyclo-octadepsipeptide antihelminthique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 382173 A
- DE 4317432 A
- DE 4317457 A
- DE 4317458 A
- EP 634408 A
- EP 718293 A
- EP 872481 A
- EP 685469 A
- EP 626375 A
- EP 664297 A
- EP 669343 A
- EP 787141 A
- EP 865498 A
- EP 903347 A
- EP OS382173 A
- WO 9319053 A
- WO 2007009606 A


### In der Beschreibung aufgeführte Nicht-Patentliteratur


- **GREIF G ; HARDER A ; HABERKORN A.** *chemotherapeutic approaches to protozoa: Caccidiae - current level of knowledge and outlook,* 2001
- **HARDER A ; GREIF G. ; HABERKORN A.** Chemotherapeutic approaches to protozoa: Kinetoplastida - current level of knowledge and outlook. *Parasitol Res,* 2001, vol. 87, 778-780
- **ADAM R.D.** Biology of Giardia lamblia. *Clinical Microbiology Reviews,* Juli 2001, 447-475
- **MARSHALL MM ; NAUMOVITZ D. ; ORTEGA Y. ; STERLING CR.** Waterborne Protozoan Pathogens. *Clinical Microbiology Reviews,* Januar 1997, 67-85
- **ROXSTRÖM-LINDQUIST, K. ; PALM D. ; REINER D. ; RINGQVIST E. ; SVÄRD SG.** Giardia immunity - an update. *Trends in Parasitology,* 2006, vol. 22 (1), 26-31
- **BECKMANN L.** Mucosal defences against Giardia. *Parasite Immunology,* 2003, vol. 25, 259-270
- **HÜLSMEIER A.J. ; KÖHLER P.** Giardia duodenalis: direct experimental evidence fort he absence of a glycosylphosphatidylinositol anchor in a variant surface protein. *Experimental Parasitology,* 2005, vol. 109, 49-52
- **KIRKPATRICK, CE ; FARRELL JP.** Feline giardiasis: observations on natural and induced infections. *Amer. J. Vet. Res.,* 1984, vol. 45, 2182-2188
- **ZIMMER JF ; BURRINGTON DB.** Comparison of four protocols fort the treatment ofcanine giardiasis. *J. Amer. Anim. Hosp. Ass.,* 1986, vol. 22, 168-172
- **ABBITT B ; HUEY RL ; EUGSTER AK ; SYLER J.** Treatment of giardiasis in adult greyhounds, using ipronidazole-medicated water. *J. Amer. Vet. Med. Ass.,* 1986, vol. 188, 67-69
- **WRIGHT JM. ; DÜNN L.A. ; UPCROFT P. ; UPCROFT J.A.** Efficacy of antigiardial drugs. *Expert Opin. Drug Saf.,* 2003, vol. 2 (6), 529-541
- **ESCOBEDO A.A ; CIMERMAN S.** Giardiasis: a pharmacotherapy review. *Expert Opin. Pharmacother,* 2007, vol. 8 (12), 1885-1902
- **HARDER A. et al.** Cyclooctadepsipeptides - an anthelmintically active class of compounds exhibiting a novel mode of action. *Int. J. Antimicrobial Agents.,* 2003, vol. 22, 318-331
- Giardiasis, Man; Encyclopedic Reference of Parasitology, Diseases, treatment, Therappy. Springer-Verlag, 2001, 234-235
- **HEWLETT, E.L. ; ANDREWS, J.S.JR. ; RUFFIER, J. ; SCHAEFER III, F.W.** experimental Infection of Mongroel Dogs with Giardia lamblia Cysts and Cultured Trophozoites. *The Journal of infectious diseases,* 1982, vol. 145 (1), 89-93